(19) Europäisches Patentamt European Patent Office Office européen des brevets

(11) **EP 2 500 755 A2**

(12) **EUROPEAN PATENT APPLICATION**

(43) Date of publication:
**19.09.2012 Bulletin 2012/38**

(51) Int Cl.:
**G02B 6/26** *(2006.01)*   **G02B 6/38** *(2006.01)*
**A61B 1/00** *(2006.01)*

(21) Application number: **12159136.6**

(22) Date of filing: **13.03.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.03.2011 JP 2011059406**

(71) Applicant: **Fujifilm Corporation**
**Minato-ku**
**Tokyo 106-8620 (JP)**

(72) Inventors:
• **Yoshida, Koji**
  **Kanagawa, 258-8538 (JP)**
• **Kasamatsu, Tadashi**
  **Kanagawa, 258-8538 (JP)**
• **Yoshihiro, Tatsuya**
  **Kanagawa, 258-8538 (JP)**

(74) Representative: **Klunker . Schmitt-Nilson . Hirsch**
**Patentanwälte**
**Destouchesstrasse 68**
**80796 München (DE)**

(54) **Optical connector and endoscope system**

(57) Provided is an optical connector including: an SI-type light source side optical fiber which is disposed on the light source side and an SI-type light receiving side optical fiber which is disposed on the light receiving side. Both optical fibers are optically coupled to each other by disposing an end surface of the light source side optical fiber and an end surface of the light receiving side optical fiber so as to face each other. The light source side optical fiber and the receiving side optical fiber are attachable to and detachable from each other. The light source side optical fiber includes a taper portion in which the diameter of the core portion increases toward the end surface of the light source side optical fiber.

FIG. 1A

## Description

## BACKGROUND OF THE INVENTION

1. **Field of the Invention**
The present invention relates to an optical connector, and particularly, to an optical connector that optically couples a light source side optical fiber and a light receiving side optical fiber to each other and an endoscope system that uses the same.

2. **Description of the Related Art**
Hitherto, as a non-contact type optical connector, there is a known optical connector that uses an optical fiber with a core portion having a refractive index distribution (which is also simply referred to as a GI-type optical fiber) or an optical fiber with a core portion having a constant refractive index (which is simply referred to as an SI-type optical fiber). In a GI collimator which is an optical connector using the GI-type optical fiber, conspicuous wavelength dependency occurs in the back focal distance, and light transmission efficiency considerably degrades with an increase in wavelength range of passing light. For this reason, the GI collimator is usually used to allow monochromatic light to pass therethrough.

Further, there is a known method of causing light with a wavelength range wider than that of monochromatic light, for example, light with different wavelengths in an infrared region to pass through the GI collimator (see JP2006-524845A).

Further, there is a known light propagating device in which a laser output from a semiconductor laser element concentrates on a convex lens and a large diameter side end surface of an optical fiber formed in a taper shape is disposed at the laser concentration point so that the laser input from the large diameter side is propagated through the small diameter side of the taper portion (see JP2006-309146A).

## SUMMARY OF THE INVENTION

However, the GI collimator through which light with different wavelengths in the infrared region passes may not fundamentally solve degradation in the light transmission efficiency with an increase in the wavelength range of the passing light. For example, in a case where white light with the wider wavelength range passes through the GI collimator, the light transmission efficiency greatly degrades with respect to a specific wavelength. Further, the light propagating device in which the taper-shaped optical fiber is installed at the light receiving side may be applied to a detachable optical connector. However, since there is a need to use a lens that suppresses occurrence of chromatic aberration in order to solve the wavelength dependency of the light transmission efficiency, there is a problem in that the size and the cost of the device increase.

The present invention has been made in view of the above-mentioned problems and an object of the present invention is to provide an optical connector capable of suppressing degradation in the light transmission efficiency caused by an increase in wavelength range of passing light without causing an increase in cost and size of a device and an endoscope system using the same.

According to an aspect of the present invention, there is provided an optical connector including: an SI-type light source side optical fiber that is disposed on a light source side and an SI-type light receiving side optical fiber that is disposed on a light receiving side. Both optical fibers are optically coupled to each other by disposing the end surfaces of the light source side optical fiber and the light receiving side optical fiber so as to face each other. Further, the light source side optical fiber and the light receiving side optical fiber are attachable to and detachable from each other. Moreover, the light source side optical fiber is an up-tapered optical fiber which has a taper portion in which the diameter of the core portion increases toward the end surface of the light source side optical fiber.

Furthermore, the SI-type optical fiber means a step-index-type optical fiber. That is, the core portion of the light source side optical fiber and the core portion of the light receiving side optical fiber have the same refractive index so that there is no refractive index distribution. Accordingly, the light which is propagated inside the respective core portions is propagated inside the respective optical fibers while being reflected in the outer wall surfaces of the core portions.

Further, the attachable and detachable state means a state in which the positional relationship between both optical fibers is fixed so that both optical fibers facing each other are maintained in an optically coupled state or a state in which both optical fibers are separated from each other so that both optical fibers do not face each other.

Further, the "taper portion in which the diameter of the core portion increases toward the end surface of the light source side optical fiber" indicates that any region in the outer wall surface (the reflection surface) of the core portion which forms the taper portion forms the outer wall surface (the reflection surface) so that the light which is propagated in the direction perpendicular to the optical axis inside the core portion is reflected toward the side where the diameter of the core increases (toward the end surface where the light is output). That is, the taper portion is not limited to the case where the taper portion is formed in a conical shape, and may be formed in a polygonal pyramid shape or a shape formed by the combination of an adjustable surface or a polyhedron. Furthermore, the optical axis may be set as the center axis which passes through the gravity center position of the cross-section of the core portion. Further, the cross-section of the core portion may be formed as a cross-section which is cut in a plane perpendicular to the direction in which the core portion extends.

In the taper portion of the light source side optical fiber, the center axis which passes through the gravity center position of the cross-section of the core portion may be formed in a linear shape.

The light receiving side optical fiber may be an up-tapered optical fiber which includes a taper portion in which the diameter of the core portion increases toward the end surface of the light receiving side optical fiber.

Furthermore, the "taper portion in which the diameter of the core portion increases toward the end surface of the light receiving side optical fiber" indicates that any region in the outer wall surface (the reflection surface) of the core portion forming the taper portion forms the outer wall surface (the reflection surface) so that the light which is propagated in the direction perpendicular to the optical axis inside the core portion is reflected toward the side where the diameter increases (toward the end surface where the light is input). That is, the taper portion is not limited to the case where the taper portion is formed in a conical shape, and may be formed in a polygonal pyramid shape or a shape formed by the combination of an adjustable surface or a polyhedron. Furthermore, the optical axis may be set as the center axis which passes through the gravity center position of the cross-section of the core portion. Further, the cross-section of the core portion may be formed as a cross-section which is cut in a plane perpendicular to the direction in which the core portion extends.

In the taper portion of the light source side optical fiber, the center axis which passes through the gravity center position of the cross-section of the core portion may be formed in a linear shape.

In the light receiving side optical fiber, the cross-section of the core portion may have the same shape and size.

In the optical connector, the end surface of the light source side optical fiber and the end surface of the light receiving side optical fiber may be disposed so as to face each other in a non-contact state while both optical fibers are optically coupled to each other.

Light with two different types or more of wavelengths may pass through the optical connector.

White light may pass through the optical connector.

In the optical connector, the area of the core portion in the end surface of the light receiving side optical fiber may be larger than the area of the core portion in the end surface of the light source side optical fiber.

In the optical connector, the end surface of the core portion of the light receiving side optical fiber and the end surface of the core portion of the light source side optical fiber may be disposed so as to face each other without excess or insufficient overlap thereof in a state where both optical fibers are optically coupled to each other.

Furthermore, "overlapping without excess or insufficient overlap" is not limited to the case where both optical fibers completely overlap each other without any excess or insufficient overlap and means the case where 90% or more of areas thereof overlap each other.

The light source side optical fiber may be desirable in the range of the conditional equation (1): $0.5 \leq L1/\varepsilon1$ and be more desirable in the range of the conditional equation (1A): $2.6 \leq L1/\varepsilon1$. Here, L1 shows the length (mm) of the taper portion of the light source side optical fiber and $\varepsilon1$ shows the taper ratio of the light source side optical fiber. Here, the taper ratio $\varepsilon1$ is a value which may be obtained by the equation of $\varepsilon1 =$ (the maximal core diameter of the taper portion in the light source side optical fiber/the minimal core diameter of the taper portion in the light source side optical fiber).

The light receiving side optical fiber may be desirable in the range of the conditional equation (2): $0.5 \leq L2/\varepsilon2$ and more desirable in the range of the conditional equation (2A): $2.6 \leq L2/\varepsilon2$. Here, L2 shows the length (mm) of the taper portion of the light receiving side optical fiber and $\varepsilon2$ shows the taper ratio of the light receiving side optical fiber. Here, the taper ratio $\varepsilon2$ is a value which may be obtained by the equation of $\varepsilon2 =$ (the maximal core diameter of the taper portion in the light receiving side optical fiber/the minimal core diameter of the taper portion in the light receiving side optical fiber).

According to another aspect of the present invention, there is provided an endoscope system including: the optical connector; a light source; and an endoscope body. Futher a light flux output from the light source may be transmitted to the endoscope body through the optical connector so that the light flux is output from the endoscope body.

According to the optical connector of the present invention and the endoscope system using the same, in the optical connector in which the light output side end surface as the end surface on the light output side of the core portion of the light source side optical fiber is optically coupled to the light input side end surface as the end surface on the light input side of the core portion of the light receiving side optical fiber while they face each other, the light source side optical fiber is formed in a taper shape in which the diameter of the core portion increases as it moves toward the light output side end surface. Accordingly, light may highly efficiently pass through the optical connector without causing an increase in cost or size of the device and degradation in the light transmission efficiency (coupling efficiency) with an increase in the wavelength range of the passing light.

That is, since the core portion of the light source side optical fiber is formed in a taper shape as described above, the angle of the light beam reflected in the outer wall surface of the core portion with respect to the extension direction of the core portion (that is, with respect to the optical axis) may be further decreased, and hence the divergence angle (also referred to as the diffusion angle) of the light flux which is output from the light output side end surface of the core portion may be decreased. Thus, the angle of the light beam which is input to the light receiving side optical fiber with respect to the extension direction of the light receiving side optical fiber (with respect to the optical axis) when both optical fibers are optically coupled to each other may be further decreased, and hence the amount of the light which is input to a portion other than the core portion of the light receiving side optical fiber may be suppressed. Accordingly, light may highly efficiently pass through the optical connector.

In addition, since the light which passes through the optical connector may be transmitted only by the reflecting action instead of the refracting action such as the GI collimator, even when the wavelength range of the passing light increases by changing the wavelength of the light or increasing the number of types of wavelengths, the light transmission efficiency may not be degraded.

Further, since the core portion of the light source side optical fiber is formed in a taper shape so that the area of the light output side end surface of the core portion increases, it is possible to decrease the energy density of the light which passes through the light output side end surface of the core portion. Thus, it is possible to obtain an effect in which adsorption of contaminants on the light output side end surface is suppressed. Furthermore, the "effect of suppressing the adsorption of contaminants" may be referred to in JP2006-309146A.

Further, since the small diameter side diameter of the tapered core portion may be decreased even when the large diameter side diameter of the tapered core portion of the light source side optical fiber (the diameter of the light output side end surface of the core portion) increases, it is possible to thin the light transmission portion as a whole and ensure the flexibility thereof.

Furthermore, when the laser is used as the light source, the laser which is output from the light source and is input to the light source side optical fiber is output from the light output side end surface of the tapered core portion (the large diameter side end surface), which may reduce the light intensity of the laser at the viewing angle. Here, it is advantageous that the viewing angle of the light output side end surface is large when the laser class of the device is determined. That is, a laser with a larger light intensity in the same class may be used.

## BRIEF DESCRIPTION OF THE DRAWINGS

Fig. 1A is a cross-sectional view illustrating a state where optical connectors according to an embodiment of the present invention are connected to each other so that they are optically coupled to each other.

Fig. 1B is a cross-sectional view illustrating a state where the optical connectors are separated from each other so that the optically coupled state is released.

Fig. 2A is a diagram illustrating an optical connector of which the gradient of a taper portion of a light receiving side optical fiber is gentle.

Fig. 2B is a diagram illustrating an optical connector of which the taper angle of a light receiving side optical fiber is set to be 0 so that the diameter of the entire core portion is large.

Fig. 2C is a diagram illustrating an optical connector of which the gradient of a taper portion of a light receiving side optical fiber is gentle and the diameter of a light input side end surface is large.

Fig. 2D is a diagram illustrating an optical connector of which the gradient of a taper portion of a light receiving side optical fiber is steep and the diameter of a light input side end surface is large.

Fig. 3 is a diagram illustrating an appearance of a light beam which passes through a core portion of a light source side optical fiber corresponding to a subject of a computer simulation.

Fig. 4 is a diagram illustrating a relationship between the taper ratio of a light source side optical fiber and the diffusion angle of a light flux output from a taper portion with this taper ratio.

Fig. 5 is a diagram illustrating an appearance in which an optical fiber with a taper portion is formed.

Fig. 6 is a diagram comparatively illustrating the light intensity distributions of the light fluxes which are output from a straight optical fiber and a tapered optical fiber.

Fig. 7A is a diagram illustrating an appearance in which the light source side optical fiber and the light receiving side optical fiber both adopt a straight optical fiber and are optically coupled to each other.

Fig. 7B is a diagram illustrating an appearance in which the light source side optical fiber and the light receiving side optical fiber both adopt a tapered optical fiber and are optically coupled to each other.

Fig. 7C is a diagram illustrating an appearance in which the light source side optical fiber adopts a tapered optical fiber, the light receiving side optical fiber adopts a straight optical fiber, and then they are optically coupled to each other.

Fig. 8 is a diagram illustrating a relationship between a separation distance and light transmission efficiency when both optical fibers are optically coupled to each other.

Fig. 9 is a diagram illustrating the simulation result of the light intensity distribution of a light flux which is output when only the taper angle of the light source side optical fiber is changed.

Fig. 10 is a diagram comparatively illustrating measurement values of the light intensity distributions of the light fluxes which are output from the tapered optical fiber and the straight optical fiber.

Fig. 11 is a diagram relating to the light source side optical fiber and illustrating the simulation result of the diffusion angle of the light flux which changes in response to a change in the length of the taper portion.

Fig. 12 is a diagram illustrating the simulation result of the optical path of the light beam which passes through the light source side optical fiber.

Fig. 13 is a diagram illustrating the simulation result in which the length of the taper portion is fixed and which shows the relationship between the taper ratio and the light transmission efficiency.

Fig. 14 is a diagram illustrating the simulation result of the optical path of the light beam which passes through the optically coupled optical fiber.

Fig. 15 is a diagram illustrating an endoscope system that uses only a laser source as an illumination light source.

Fig. 16 is a diagram illustrating an endoscope system that uses both a laser source and a xenon lamp as an illumination light source.

Fig. 17 is a diagram illustrating an endoscope system that includes a fluorescent illumination laser source as an illumination light source.

## DESCRIPTION OF THE PREFERRED EMBODIMENTS

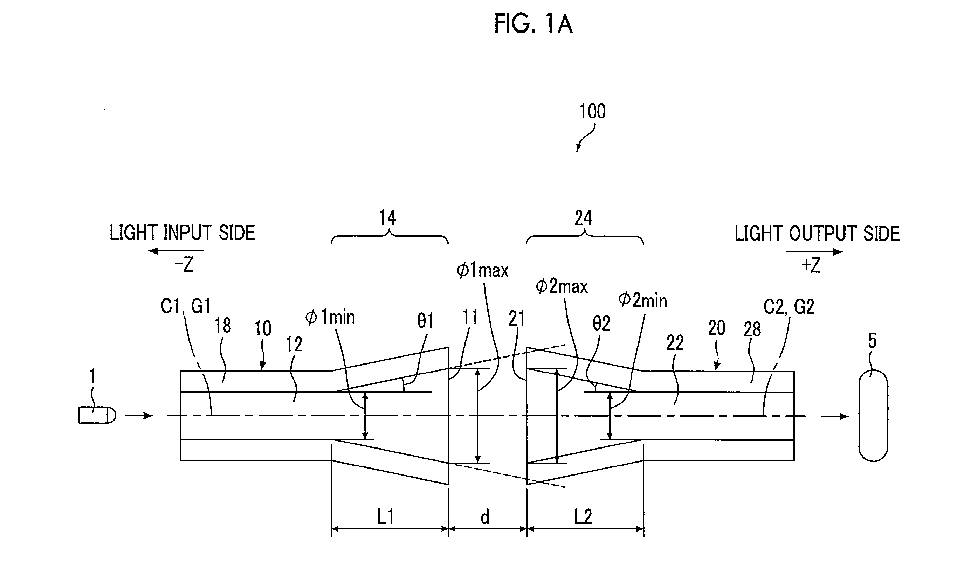

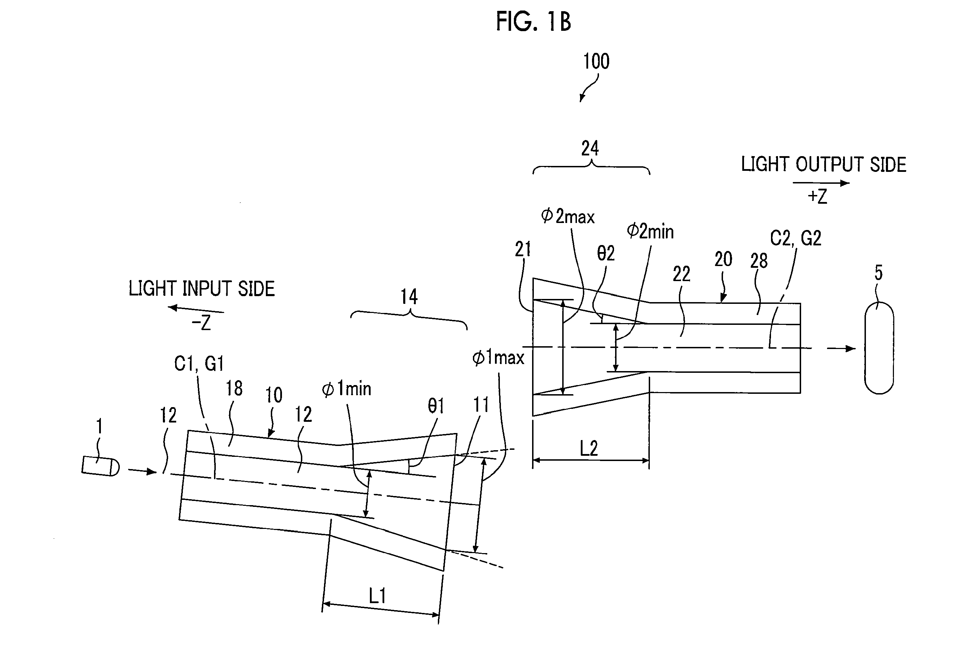

Hereinafter, an exemplary embodiment of the present invention will be described by referring to the drawings. Figs. 1A and 1B are cross-sectional views schematically illustrating a configuration of an optical connector of the present invention. Fig. 1A is a cross-sectional view illustrating a state where the optical connectors of the present invention are connected to each other so that they are optically coupled to each other. Fig. 1B is a cross-sectional view illustrating a state where the optical connectors of the present invention are separated from each other so that the optically coupled state is released.

All the optical connectors which will be described in the embodiment of the present invention adopt a step-index-type (SI-type) optical fiber as a light source side optical fiber and a light receiving side optical fiber.

An optical connector 100 according to the embodiment of the present invention shown in Figs. 1A and 1B includes: a light source side optical fiber 10 which is an optical fiber disposed on the light source side (on the -Z arrow side in the drawing) and a light receiving side optical fiber 20 which is an optical fiber disposed on the light receiving side (on the +Z arrow side in the drawing), and is configured to optically couple both optical fibers 10 and 20 to each other by disposing an end surface 11 of the light source side optical fiber 10 and an end surface 21 of the light receiving side optical fiber 20 so as to face each other.

Furthermore, the light source side optical fiber 10 which is disposed on the light source side is a step-index-type optical fiber, and the light source side optical fiber 10 is optically coupled to the light which is output from the light source 1. Further, the light receiving side optical fiber 20 which is disposed on the light receiving side is also a step-index-type optical fiber, and the light receiving side optical fiber 20 is optically coupled to an illumination unit 5. When the light source side optical fiber 10 and the light receiving side optical fiber 20 are optically coupled to each other, the light which is output from the light source 1 is transmitted to the illumination unit 5 through the optical connector 100. The optical connector 100 optically couples the optical path formed by the light source side optical fiber 10 to the optical path formed by the light receiving side optical fiber 20 so that the light which is output from the light source 1 and passes through the light source side optical fiber 10 is propagated to the light receiving side optical fiber 20 and is transmitted to the illumination unit 5. That is, the optical connector 100 optically couples both optical fibers 10 and 20 to each other so that the light output from the light source side optical fiber 10 is input to the light receiving side optical fiber 20.

The optical connector 100 enables the attachment and detachment operation between the light source side optical fiber 10 and the light receiving side optical fiber 20 (enables the attachment and detachment of the light receiving side optical fiber 20 with respect to the light source side optical fiber 10). That is, the optical connector 100 may fix the positional relationship between both optical fibers 10 and 20 so that both optical fibers 10 and 20 facing each other are maintained in an optically coupled state (see Fig. 1A) or may separate the light source side optical fiber 10 and the light receiving side optical fiber 20 from each other so that both optical fibers 10 and 20 do not face each other (see Fig. 1B). Here, in the optically coupled state, the end surface 11 of the light source side optical fiber 10 and the end surface 21 of the light receiving side optical fiber 20 may be separated from each other. At this time, an air layer may be formed therebetween or may not be formed therebetween.

The light source side optical fiber 10 includes a taper portion 14 in which the diameter of a core portion 12 increases as it moves from the light source side toward the optically coupled end surface 11.

It is desirable that the light source side optical fiber 10 is in the range of the conditional equation (1): $0.5 \leq L1/\varepsilon1$, where L1 shows the length of the taper portion 14 (the length in the direction of the optical axis C1) and $\varepsilon1$ shows the taper ratio.

Furthermore, the taper ratio $\varepsilon1$ is a value which may be obtained by the equation of $\varepsilon1 = \phi1max/\phi1min$. Here, $\phi1max$ shows the maximal diameter of the core portion 12 (the maximal core diameter) in the taper portion 14, and $\phi1min$ shows the minimal diameter of the core portion 12 (the minimal core diameter) in the taper portion 14.

In addition, it is desirable that the light source side optical fiber 10 is in the range of the conditional equation (1A): $2.6 \leq L1/\varepsilon1$.

On the other hand, the light receiving side optical fiber 20 includes a taper portion 24 in which the diameter of the core portion 22 increases toward the optically coupled end surface 21. That is, the light receiving side optical fiber 20 includes the taper portion 24 in which the diameter of the core portion 22 decreases as it moves from the optically coupled end surface 21 toward the light receiving side (toward the +Z arrow in the drawing) (as it moves toward the illumination unit 5).

It is desirable that the light receiving side optical fiber 20 is also in the range of the conditional equation (2): $0.5 \leq L2/\varepsilon2$ and the conditional equation (2A): $2.6 \leq L2/\varepsilon2$, where L2 shows the length of the taper portion 24 in the direction of the optical axis C2 and $\varepsilon2$ shows the taper ratio in the same way as described above.

The taper angle $\theta1$ which is shown in Figs. 1A and 1B is an angle which is formed between the outer wall surface of the taper portion 14 in the core portion 12 and the optical axis C1, and is an angle which is determined by the length L1 of the taper portion 14, the maximal core diameter $\phi1max$, and the minimal core diameter $\phi1min$.

Further, the taper angle $\theta2$ which is shown in Figs. 1A and 1B is an angle which is formed between the outer wall surface of the taper portion 24 in the core portion 22 and the optical axis C2, and is an angle which is determined by the length L2 of the taper portion 24, the maximal core diameter $\phi2max$, and the minimal core diameter $\phi2min$.

Furthermore, the light source side optical fiber 10 has a configuration in which a clad portion 18 is disposed on the outer periphery of the core portion 12, and the light receiving side optical fiber 20 also has a configuration in which a clad portion 28 is disposed on the outer periphery of the core portion 22. Then, the optical fibers have a configuration in which light is reflected in the outer wall surface of the core portion 12 (22) which is a boundary between the core portion 12 (22) and the clad portion 18 (28) with different refractive indexes. Here, when the values of the taper angles $\theta1$ and $\theta2$ decreases, the boundary with respect to the optical axis C1 (C2), that is, the gradient of the outer wall surface of the core portion 12 (22) becomes gentle.

Further, the optical connector 100 has a configuration in which the optical axis C1 of the light source side optical fiber 10 matches the optical axis C2 of the light receiving side optical fiber 20 when both optical fibers 10 and 20 are optically coupled to each other. That is, when the optical fibers are optically coupled to each other, the center axis G1 of the core portion 12 of the light source side optical fiber 10 matches the center axis G2 of the core portion 22 of the light receiving side optical fiber 20.

Further, the optical connector 100 has a configuration in which the end surface 11 of the light source side optical fiber 10 and the end surface 21 of the light receiving side optical fiber 20 are disposed so as to face each other while being separated from each other by a separation distance d as described above when both optical fibers 10 and 20 are optically coupled to each other.

Since the optical connector 100 adopts a configuration in which step-index-type (SI-type) optical fibers with core portions having a constant refractive index are optically coupled to each other, the wavelength dependency of the light transmission efficiency may be reduced during the optical coupling operation. Accordingly, light with a large wavelength range, for example, white light may pass through the optical connector without causing color degradation.

In addition, according to the optical connector 100, the divergence angle (which is also called the diffusion angle) of the light flux output from the light source side optical fiber may be decreased without using a refracting optical system such as a lens and the light receiving side optical fiber includes the taper portion. Accordingly, since degradation in the light transmission performance caused by the positional deviation of the light receiving side optical fiber in a direction perpendicular to the optical axis with respect to the light source side optical fiber may be reduced, degradation in the light transmission efficiency caused by the attachment and detachment between both optical fibers may be suppressed.

Furthermore, the separation distance d which is a gap between the end surface 11 of the light source side optical fiber 10 and the end surface 21 of the light receiving side optical fiber 20 which are disposed so as to face each other when both optical fibers 10 and 20 are optically coupled to each other may be, for example, equal to or larger than 0.5 mm and equal to or smaller than 3 mm. Further, the taper length L which is the length of the taper portion may be, for example, equal to or larger than 2 mm and equal to or smaller than 10 mm.

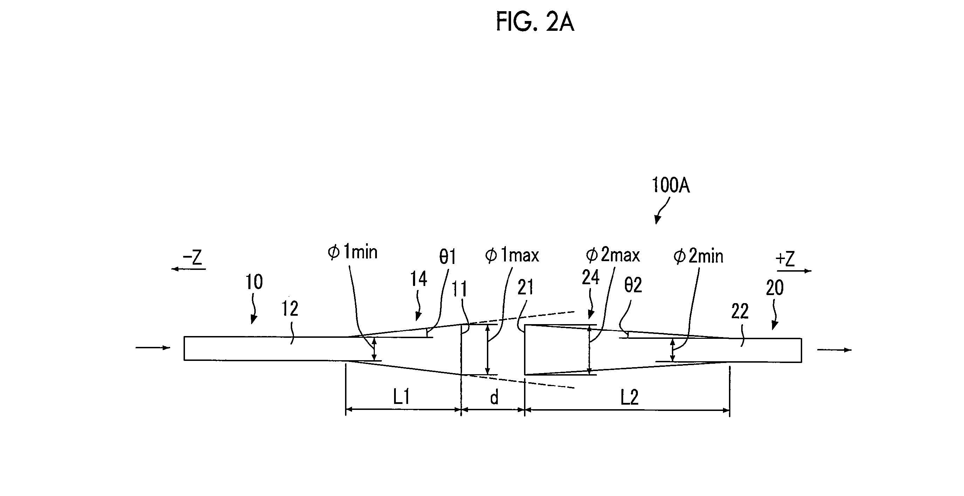

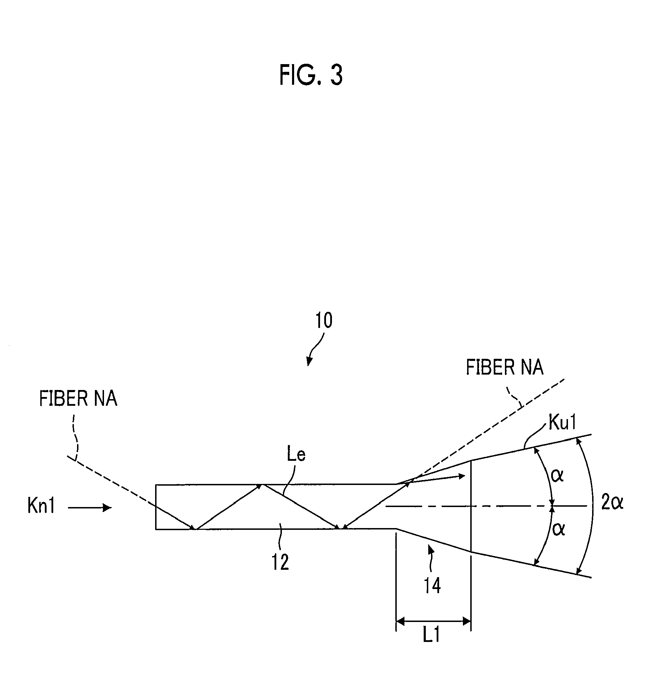

Figs. 2A to 2D illustrate the modified examples of the optical connector of the present invention, where the clad portions 18 and 28 of the optical fiber are not shown, but only the core portions 12 and 22 are shown. Furthermore, the same applies to the optical fiber shown in the drawings after Fig. 3, and the clad portion is not shown, but only the core portion is shown.

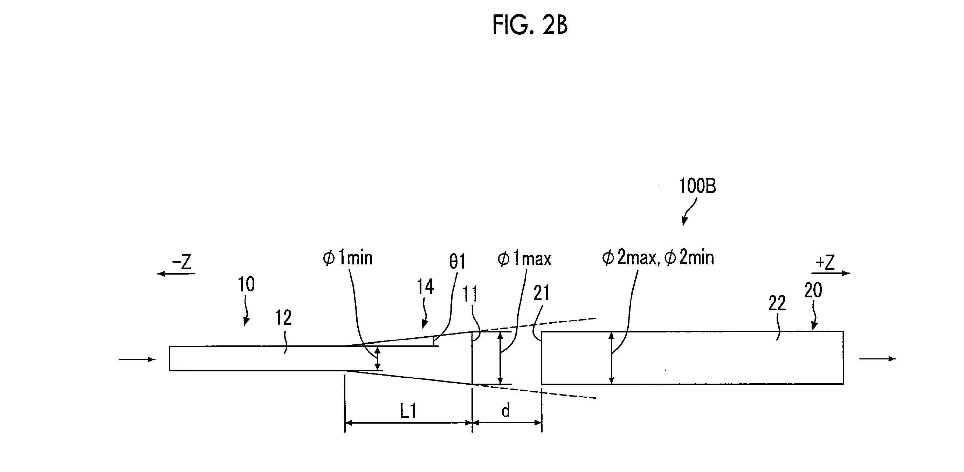

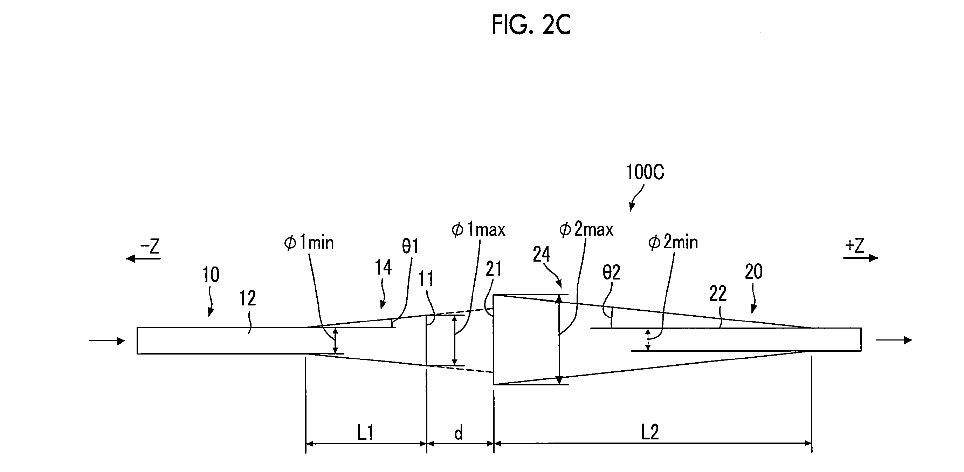

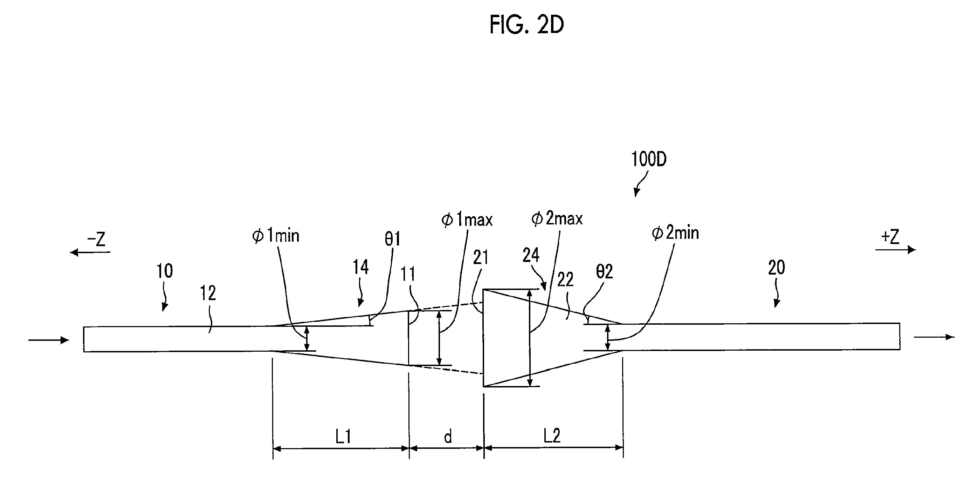

Fig. 2A is a diagram illustrating an optical connector 100A in which the gradient of a taper portion of a light receiving side optical fiber is gentle. Fig. 2B is a diagram illustrating an optical connector 100B in which a taper portion is not formed in a light receiving side optical fiber so that the diameter of the entire core portion is thick and constant. Fig. 2C is a diagram illustrating an optical connector 100C in which the gradient of a taper portion of a light receiving side optical fiber is gentle so that the diameter of the light input side end surface is large. Fig. 2D is a diagram illustrating an optical connector 100D in which the gradient of a taper portion of a light receiving side optical fiber is steep so that the diameter of the light input side end surface is large.

Hereinafter, the optical connectors 100A to 100D will be individually described. Furthermore, the description of the respective optical connectors is based on the condition that the light receiving side optical fiber 20 and the light source side fiber 10 both include taper portions so that they have the same shape. Herein, the same shape corresponds to a case where the maximal core diameters $\phi1max=\phi2max$, the minimal core diameters $\phi1min=\phi2min$, the taper lengths L1=L2 as the lengths of the taper portions, and the taper angles $\theta1=\theta2$ in the shape of both optical fibers. Here, the optical connector which is used as the reference is referred to as a reference optical connector.

Furthermore, the separation gap d and the respective dimensions of the light source side optical fiber 10 (the maximal core diameter φ1max, the minimal core diameter φ1min, the taper length L1, and the taper angle θ1) are the same in the respective optical connectors 100A to 100D.

<Optical connector 100A>

In the optical connector 100A of Modified example A shown in Fig. 2A, the maximal core diameter φ2max and the minimal core diameter φ2min of the light receiving side optical fiber 20 are set to be equal to those of the reference optical connector, and the length L2 of the taper portion 24 is set to be longer than that of the reference optical connector (that is, the taper angle θ2 is set to be smaller than that of the reference optical connector).

In this way, when the taper angle θ2 of the core portion 22 of the light receiving side optical fiber 20 is decreased in a state where the maximal core diameter φ1max of the light source side optical fiber 10 and the maximal core diameter φ2max of the light receiving side optical fiber 20 have the same diameter (in addition, the end surfaces of the core portions 11 and 21 overlap each other without excess or insufficient overlap), it is possible to further reduce the loss of the optical amount of the optical connector 100A when the optical fibers are optically coupled to each other. That is, when the light receiving side optical fiber is formed in the above-described shape, it is possible to further reduce the loss of the optical amount compared to the case where the light receiving side optical fiber and the light source side optical fiber are formed in the same shape.

The optical connector 100A has an advantage that a mode may be easily changed when the optical fibers are optically coupled to each other. Further, the optical connector 100A has a disadvantage that the manipulation becomes slightly difficult (the flexibility slightly degrades) because the thick portion of the light receiving side optical fiber is lengthened.

<Optical connector 100B>

In the optical connector 100B of Modified example B shown in Fig. 2B, the maximal core diameter φ2max of the light receiving side optical fiber 20 is set to be equal to that of the reference optical connector and the taper portion is not provided (the taper angle θ2=0).

In this way, when the core portion 22 of the light receiving side optical fiber 20 is set to have a constant thickness (as a straight optical fiber) in a state where the maximal core diameter φ1max of the light source side optical fiber 10 and the maximal core diameter φ2max of the light receiving side optical fiber 20 have the same diameter (in addition, the end surfaces of the core portions 11 and 21 overlap each other without excess or insufficient overlap), it is possible to further reduce the loss of the optical amount of the optical connector 100B when the optical fibers are optical coupled to each other compared to the case where the light receiving side optical fiber and the light source side optical fiber have the same shape.

The optical connector 100B has an advantage that the attenuation of the light amount of the light passing through the light receiving side optical fiber may be reduced. Further, the optical connector 100B has a disadvantage that the manipulating becomes difficult because the entire light receiving side optical fiber has a large diameter (the flexibility degrades).

< Optical connector 100C>

In the optical connector 100C of Modified example C shown in Fig. 2C, the length L2 of the taper portion 24 and the maximal core diameter φ2max is set to be larger than those of the reference optical connector without changing the taper angle θ2 and the minimal core diameter φ2min.

In this way, when the maximal core diameter φ2max of the light receiving side optical fiber 20 is set to be larger than the maximal core diameter φ1max of the light source side optical fiber 10, it is possible to further reduce the loss of the optical amount of the optical connector 100C when the optical fibers are optically coupled to each other. That is, since the light receiving side optical fiber 20 may receive the light which is output from the light source side optical fiber 10 without any leakage thereof (leaking light), it is possible to reduce the loss of the optical amount.

The optical connector 100C has advantages that the leakage of light (leaking light) when the optical fibers are optically coupled to each other may be reduced and a mode may be easily changed because. Further, the optical connector 100C has a disadvantage that the manipulating becomes slightly difficult (the flexibility slightly degrades) because the thick portion of the light receiving side optical fiber is lengthened.

< Optical connector 100D>

In the optical connector 100D of Modified example D shown in Fig. 2D, the maximal core diameter φ2max is set to be larger (the taper angle θ2 is set to be larger) than that of the reference optical connector while the length L2 of the taper portion 24 and the minimal core diameter φ2min are set to be equal to those.

In this way, when the maximal core diameter φ2max of the light receiving side optical fiber 20 is set to be larger than the maximal core diameter φ1max of the light source side optical fiber 10, it is possible to further reduce the loss of the optical amount of the optical connector 100D when the optical fibers are optically coupled to each other (as in the case of the optical connector 100C). On the other hand, since the taper angle θ2 is large, the loss of the optical amount inside the taper portion 24 increases.

The optical connector 100D has an advantage that the leakage of light (the leaking light) when the optical fibers are optically coupled to each other may be reduced. Further, the optical connection 100D has a disadvantage that loss when a mode changes increases.

Next, examples and the like according to the present invention will be further described.

<Computer simulation of light source side optical fiber according to BPM method>

The BPM method is a method in which the appearance of the electric field propagated inside the optical waveguide or the optical fiber is shown by computer simulation.

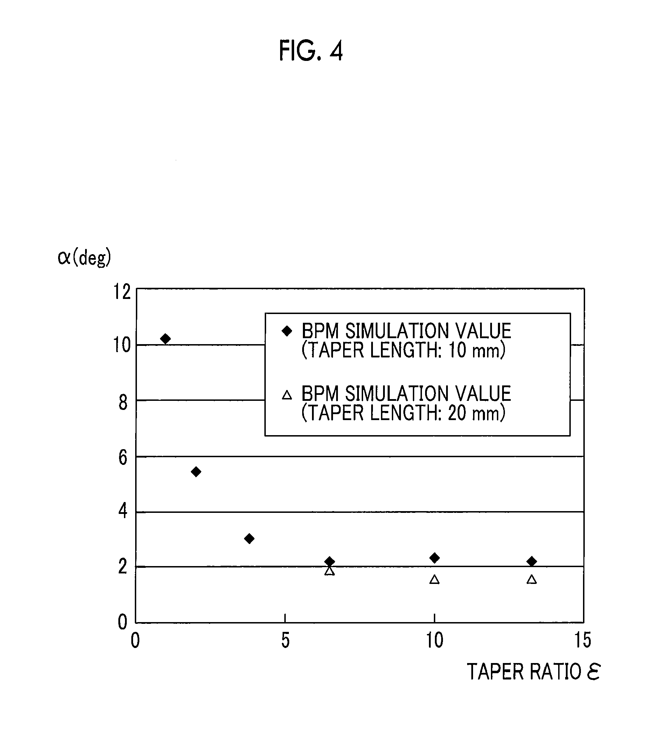

Fig. 3 is a schematic diagram illustrating the appearance of the core portion of the light source side optical fiber corresponding to the subject of the computer simulation and the light beam which passes through the core portion thereof. Further, Fig. 4 is a diagram illustrating a relationship between the taper ratio of the light source side optical fiber and the diffusion angle of the light flux output from the taper portion with this taper ratio on the coordinate plane where the horizontal axis indicates the taper ratio $\varepsilon$ and the vertical axis indicates the diffusion half angle $\alpha$ (the total angle $2\alpha$) of the light flux output through the taper portion.

Here, the FFP (far-field pattern) when the taper length L1 is 10 mm and 20 mm is calculated.

As can be understood from Figs. 3 and 4, the diffusion half angle $\alpha$ of the light flux Ku1 output from the taper portion 14 is inverse proportional to the taper ratio until the taper ratio $\varepsilon$ increases to 3.8 when the taper length is set to 10 mm. That is, it was found that the diffusion angle reduction effect was exhibited. Further, when the taper ratio $\varepsilon$ was equal to or higher 6.5, the inverse proportional relationship was not maintained, so that the lower limit of the diffusion half angle $\alpha$ was a constant such as 2°.

Furthermore, if the taper length was set to 20 mm, the lower limit of the diffusion angle was slightly improved when the taper ratio $\varepsilon$ was equal to or higher than 6.5, so that the diffusion half angle $\alpha$ was a constant such as 1.6°.

It was found that this experiment was close to the limit of the preservation of etendue because the taper ratio $\varepsilon$ was 3.8 ($\phi$1min=60 $\mu$m, $\phi$1max=230 $\mu$m).

The condition when performing the computer simulation is shown below.

In the computer simulation, the optical waveguide simulation program developed by OPTIWAVE Corporation was used.

The input light flux Kn1: Gaussian beam with spot diameter of 0.5 $\mu$m at wavelength of 780 nm
Length of straight portion: 40 mm (20 mm)
Taper length L1: 10 mm, 20 mm
Refractive index of core portion: 1.45
Refractive index of clad portion: 1.433
Refractive index of medium: 1.00
Taper shape: $\exp^{(-1)}$

Here, the numerical value in parentheses indicates a case where the taper ratio $\varepsilon$ is equal to or higher than 6.5.

Furthermore, the NA xcore diameter (the diameter of the core portion) is constant, the clad mode equal to or higher than the input side fiber NA is removed, and the NA at the light output side taper portion decreases.

Further, the trace (the optical path) of the light beam Le which is propagated inside the core portion 12 is shown in the drawing.

<Manufacturing of optical fiber with taper portion>

Hereinafter, the method of manufacturing the optical fiber with the taper portion will be described.

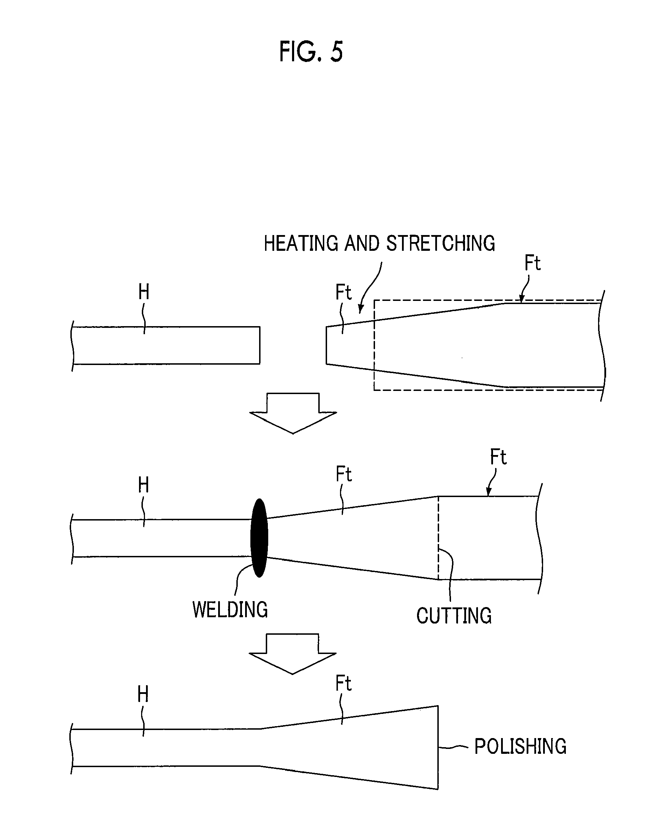

Fig. 5 is a diagram illustrating the appearance in which the optical fiber with the taper portion is formed in a manner such that the large-diameter optical fiber is heated and stretched so as to be welded to the small-diameter optical fiber. The upper-stage diagram in Fig. 5 illustrates the appearance in which the large-diameter optical fiber is heated and stretched. The middle-stage diagram illustrates the appearance in which the large-diameter optical fiber F which is heated and stretched is welded to the small-diameter optical fiber H. The lower-stage diagram illustrates the appearance in which the large diameter side of the taper portion of the welded optical fiber is cut and the end surface is polished.

In order to manufacture the optical fiber with the taper portion, a large-diameter optical fiber F and a small-diameter optical fiber H are prepared.

Here, the large-diameter optical fiber F is a straight optical fiber with NA: 0.22, a core diameter: 230 $\mu$m, and a clad diameter: 250 $\mu$m, and the material of forming the core portion is quartz.

The small-diameter optical fiber H has NA: 0.22, a core diameter: 60 $\mu$m, and a clad diameter: 80 $\mu$m, and the material of forming the core portion is quartz.

First, the large-diameter optical fiber F is heated and stretched by a ceramic heater of about 1400°, so that a taper portion Ft formed in a taper shape is provided.

Next, the core portion in the end surface of the taper portion Ft is positioned with respect to the core portion in the end surface of the small-diameter optical fiber.

Subsequently, the end surface of the taper portion in the large-diameter optical fiber is welded to the end surface of the small-diameter optical fiber.

The large diameter side of the taper portion is cut, the end surface of the taper portion integrally welded to the small-diameter optical fiber is optically polished, and then is fixed to an FC connector.

In this way, the optical fiber with the tapper portion of which the taper length L=10 mm and the taper ratio $\varepsilon$=3.8 may be obtained.

Furthermore, the core diameter or the clad diameter of the large-diameter optical fiber and the small-diameter optical fiber is not limited to the above-described case.

Since the size of the outer periphery of the general optical fiber for the FC connector is $\phi$2.5 mm, it is desirable that the maximal clad diameter is equal to or smaller than $\phi$2.5 mm.

Furthermore, the optimal value of the taper length L will be described later.

<Measurement of diffusion angle of light flux output from taper portion>

Next, the measurement of the diffusion angle of the light flux which is output from the light source side optical fiber with the taper portion will be described.

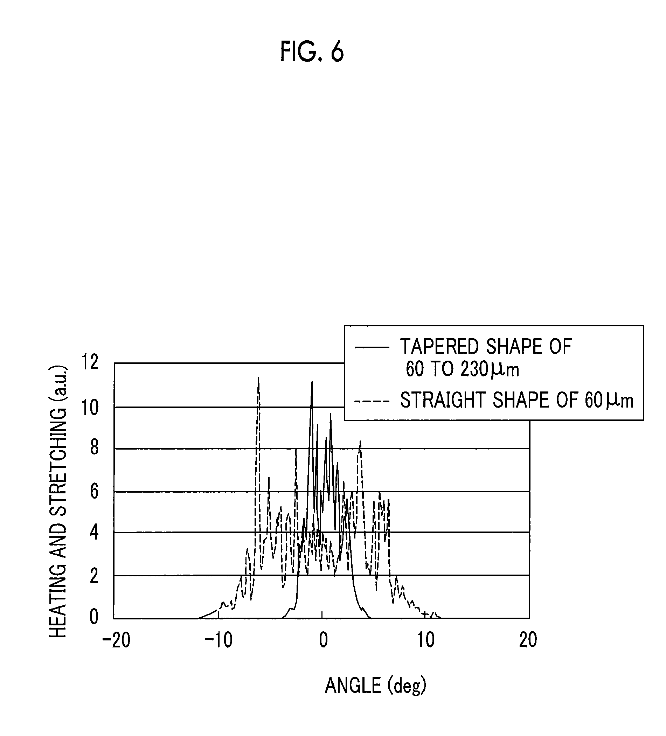

Fig. 6 is a diagram comparatively illustrating the light intensity distributions of the light fluxes which are output from the straight optical fiber and the tapered optical fiber on the coordinate plane where the horizontal axis indicates the angle formed with respect to the optical axis and the vertical axis indicates the light intensity.

The straight optical fiber has a constant core diameter of 60 $\mu$m.

In the tapered optical fiber, the thinnest core diameter of the taper portion (the minimal core diameter) is 60 $\mu$m, the thickest core diameter of the taper portion (the maximal core diameter) is 230 $\mu$m, and the taper ratio $\varepsilon$ is 3.8 ($\varepsilon$=3.8 $\approx$230/60).

As understood from Fig. 6, when NA is calculated with the diffusion angle given as the angle at which the light intensity of 5% of the peak value of the light intensity distribution is obtained, NA of the tapered optical fiber becomes 0.058, and NA of the straight optical fiber becomes 0.169, so that NA of the tapered optical fiber decreases by 1/3 compared to NA of the straight optical fiber.

<Measurement of light transmission efficiency>

Next, the measurement of the light transmission efficiency when the optical fibers are optically coupled to each other will be described.

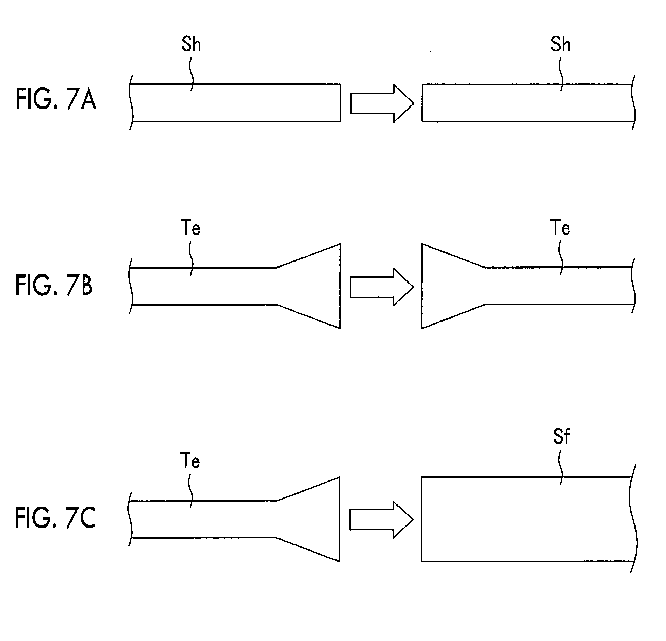

Fig. 7A is a diagram illustrating the appearance in which the light source side optical fiber and the light receiving side optical fiber are both formed as a straight optical fiber and are optically coupled to each other. Fig. 7B is a diagram illustrating the appearance in which the light source side optical fiber and the light receiving side optical fiber are both formed as a tapered optical fiber and are optically coupled to each other. Fig. 7C is a diagram illustrating the appearance in which the light source side optical fiber is formed as a tapered optical fiber, the light receiving side optical fiber is formed as a straight optical fiber, and both optical fibers are optically coupled to each other.

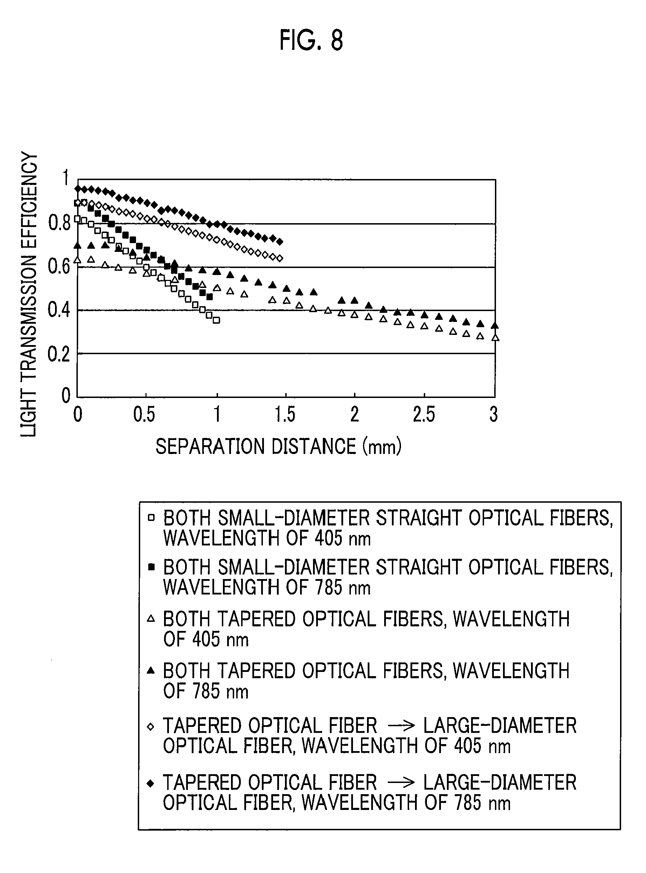

Further, Fig. 8 is a diagram illustrating a relationship between the separation distance and the light transmission efficiency when both optical fibers are optically coupled to each other on the coordinate plane where the horizontal axis indicates the separation distance d and the vertical axis indicates the light transmission efficiency E.

As the light source side optical fiber, two types were prepared. That is, the tapered optical fiber (the taper portion with a minimal core diameter of 60 $\mu$m and a maximal core diameter of 230 $\mu$m) and the small-diameter straight optical fiber (with a constant core diameter of 60 $\mu$m) were prepared.

As the light receiving side optical fiber, three types were prepared. That is, the tapered optical fiber (the taper portion with a minimal core diameter of 60 $\mu$m and a maximal core diameter of 230 $\mu$m), the small-diameter straight optical fiber (with a constant core diameter of 60 $\mu$m), and the large-diameter straight optical fiber (with a constant core diameter of 230 $\mu$m) were prepared.

Further, as the wavelength of the laser, two types were prepared. That is, laser sources which output lasers with wavelengths of 405 nm and 785 nm were prepared.

As the separation distance which is a parameter of measurement, plural separation distances were gradually set and measured from a state where the end surfaces of both optical fibers adhere to each other (the separation distance d=0) to a distance where the separation distance becomes 3 mm (d=3 mm).

The light transmission efficiency was measured by gradually changing the separation distance using a laser which passes through the optical connector that optically couples the combination of the respective optical fibers.

Furthermore, as an optical connector which is a measurement subject of light transmission efficiency, there are three types of optical connectors shown in Figs. 7A to 7C. Fig. 7A illustrates an optical connector of which the light source side is formed as a small-diameter straight optical fiber Sh and the light receiving side is formed as a small-diameter straight optical fiber Sh. Fig. 7B is a diagram illustrating an optical connector of which the light source side is formed as a tapered optical fiber Te and a light receiving side is formed as a tapered optical fiber Te. Fig. 7C is a diagram illustrating an optical connector of which the light source side is formed as a tapered optical fiber Te and the light receiving side is formed as a large-diameter straight optical fiber Sf.

As understood from Fig. 8, when the light source side is formed as the tapered optical fiber and the light receiving side is formed as the large-diameter straight optical fiber (see Fig. 7C) compared to the case where both optical fibers are formed as the small-diameter straight optical fibers (see Fig. 7A), it is found that the light transmission efficiency is high in any separation distance and any wavelength.

Further, as described above, the case where both optical fibers are formed as a small-diameter straight optical fiber is used as the reference case. Then, the light transmission efficiency is low when both optical fibers are formed as a tapered optical fiber in the state where the separation distance is in the range of 0 to 0.5 mm. However, the light transmission efficiency is high when both optical fibers are formed as a tapered optical fiber in the state where the separation distance is in the range of 0.5 to 1.0 mm.

<Optimal length of taper portion>

Hereinafter, the result which is examined for the optimal length of the taper portion formed in the light source side optical fiber will be described.

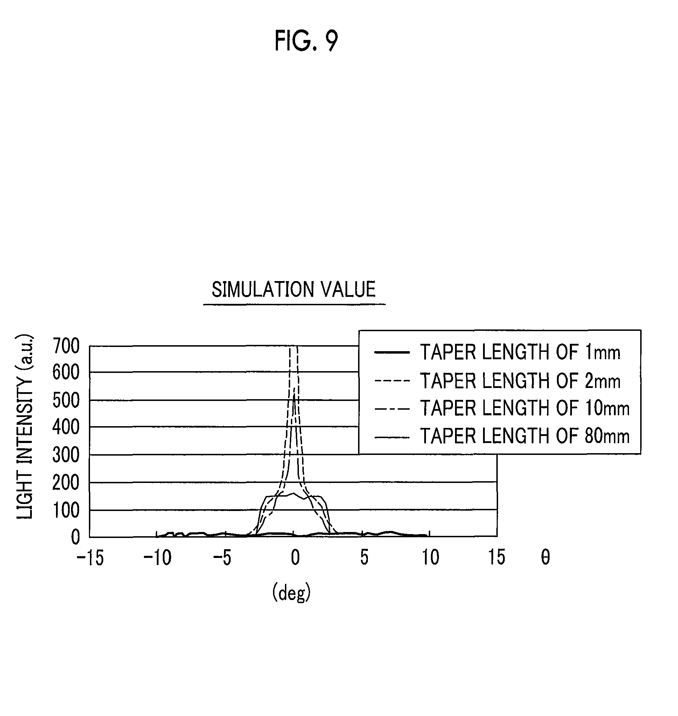

Fig. 9 is a diagram illustrating the simulation result of the light intensity distribution of the light flux output from the taper portion when only the taper angle is changed without changing the minimal core diameter and the maximal core diameter of the taper portion of the light source side optical fiber (that is, when only the taper length is changed) on the coordinate plane where the horizontal axis indicates the taper angle θ1 and the vertical axis indicates the light intensity.

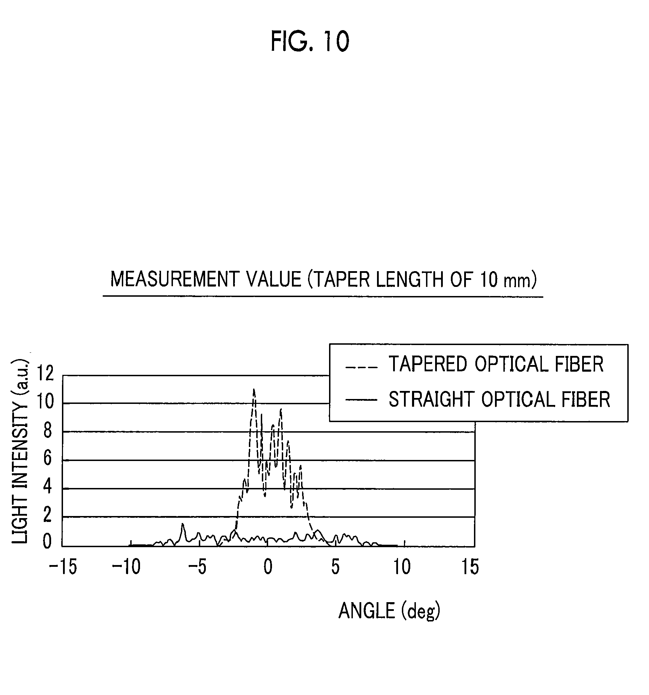

Fig. 10 is a diagram comparatively illustrating the measurement values of the light intensity distributions of the light fluxes output from the straight optical fiber and the tapered optical fiber with a taper length of 10 mm on the coordinate plane where the horizontal axis indicates the taper angle and the vertical axis indicates the light intensity. Furthermore, the core diameter of the straight optical fiber is equal to the minimal core diameter of the taper portion of the tapered optical fiber.

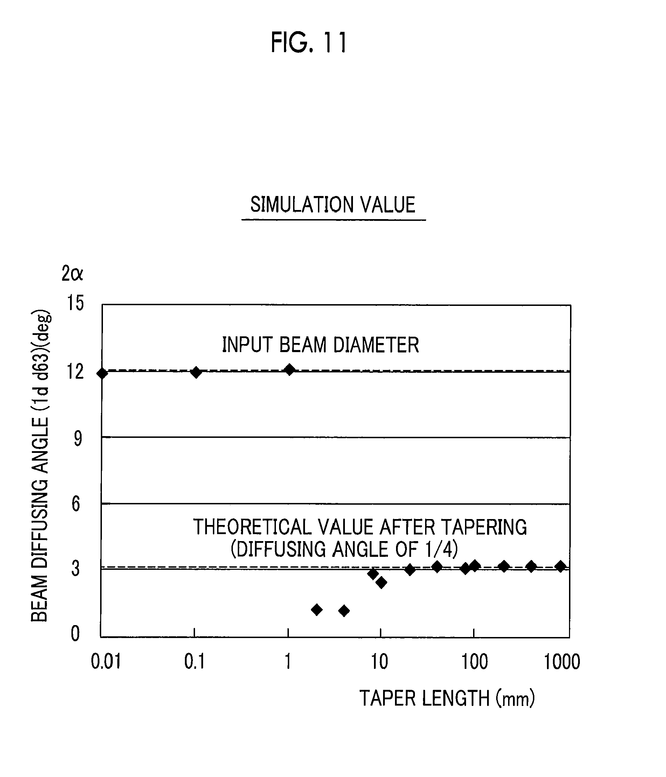

Fig. 11 is a diagram illustrating the simulation result of the diffusion angle of the light flux which changes in response to a change in the taper length on the coordinate plane where the horizontal axis indicates the taper length and the vertical axis indicates the diffusion total angle 2α of the light flux output from the taper portion.

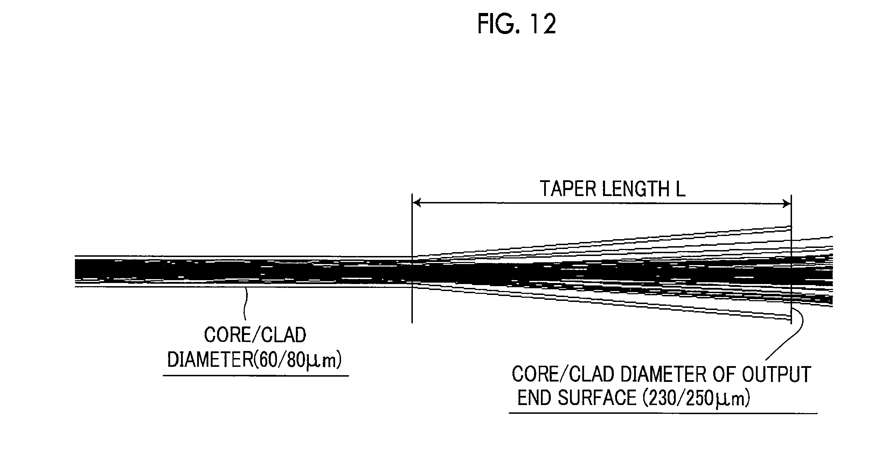

Fig. 12 is a diagram illustrating the simulation result of the trace (the optical path) of the light beam which passes through the light source side optical fiber. The simulation result which is obtained herein may be obtained by the light beam tracking simulation using the common optical design tool which is developed by ZEMAX Corporation.

In the computer simulation or the measurement, the minimal core diameter of the taper portion was set to 60 μm and the maximal core diameter was set to 230 μm. Accordingly, the taper ratio ε of the taper portion is 3.8.

Further, the input angle (the total angle) of the light flux with respect to the light source side optical fiber was set to 20° and NA of the optical fiber was calculated as 0.23. Further, the diffusion total angle 2α of the light flux shown in Fig. 11 was calculated as the angle including 63% of the light intensity in the plot shown in Fig. 9 (see the "beam diffusion angle (1D d63) (deg)" of Fig. 11).

As can be understood from the simulation results of Figs. 9 and 11, when the taper ratio ε is 3.8, the length of the taper portion may be desirably 2 mm or more and more desirably 10 mm or more. In other words, the value which is obtained by dividing the taper length by the taper ratio (the value which is obtained by the equation of the taper length/the taper ratio) may be desirably 0.5 mm or more and more desirably 2.6 mm or more.

Further, as understood from the simulation result of Fig. 9 and the measurement result of Fig. 10, the simulation result and the measurement result are substantially equal to each other.

<Optimal taper ratio>

Next, the result which is examined for the optimal taper ratio of the taper portion formed in the optical fibers which are optically coupled to each other will be described.

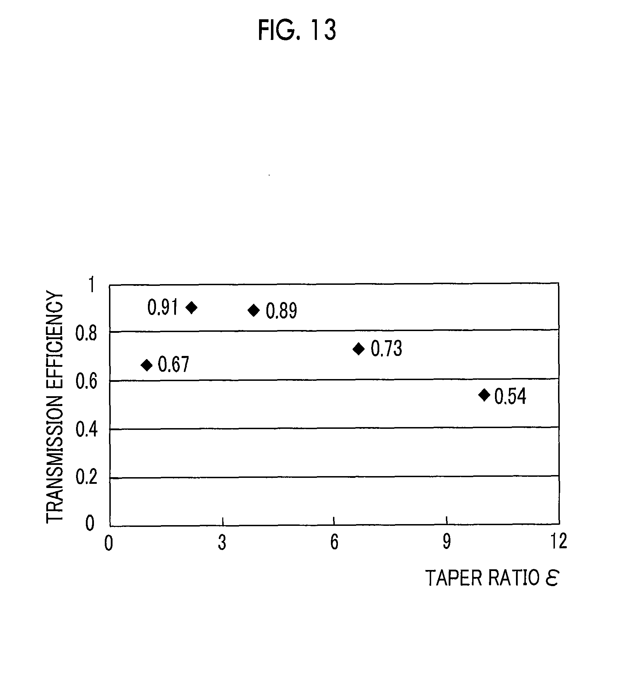

Fig. 13 is a diagram illustrating the simulation result of the relationship between the taper ratio and the light transmission efficiency when both optical fibers are optically coupled to each other with the taper length fixed to 10 mm on the coordinate plane where the horizontal axis indicates the taper ratio and the vertical axis indicates the light transmission efficiency.

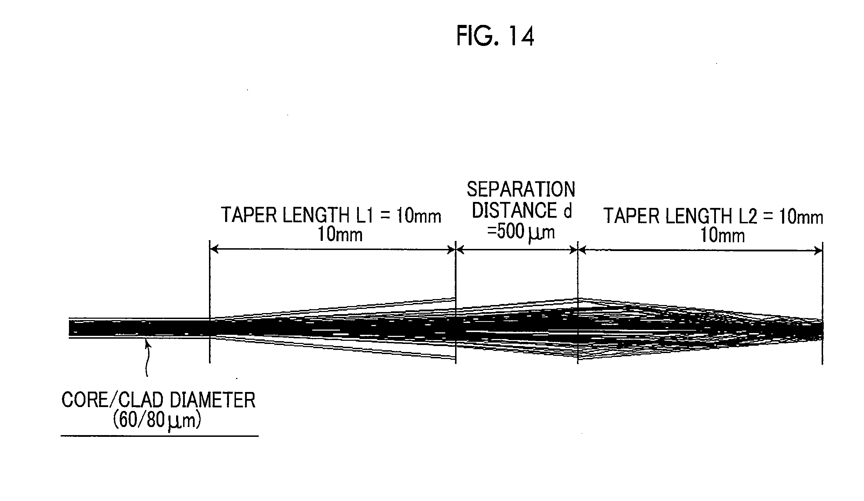

Fig. 14 is a diagram illustrating the simulation result of the trace (the optical path) of the light beam which passes through the optical fibers which are optically coupled to each other.

The simulation result which is obtained herein may be obtained by the light beam tracking simulation using the common optical design tool which is developed by ZEMAX Corporation.

As the computer simulation, in both the light source side optical fiber and the light receiving side optical fiber, the minimal core diameters φ1min and φ2min of the taper portions were set to 60 μm and the taper lengths L1 and L2 were set to 10 mm. In this state, a change in the light transmission efficiency was obtained by calculation when the sizes of the maximal core diameters φ1max and φ2max of the taper portions of both optical fibers changed together. Furthermore, the separation gap d was a constant such as 500 μm.

As understood from Fig. 13, the light transmission efficiency is 0.67 when the taper ratio is 1, and the light transmission efficiency is 0.91 when the taper ratio is 2.2. Then, the light transmission efficiency degrades as much as the amount in which the taper ratio increases.

<Application to endoscope system>

Next, a case will be described in which the optical connector of the present invention is applied to the endoscope system.

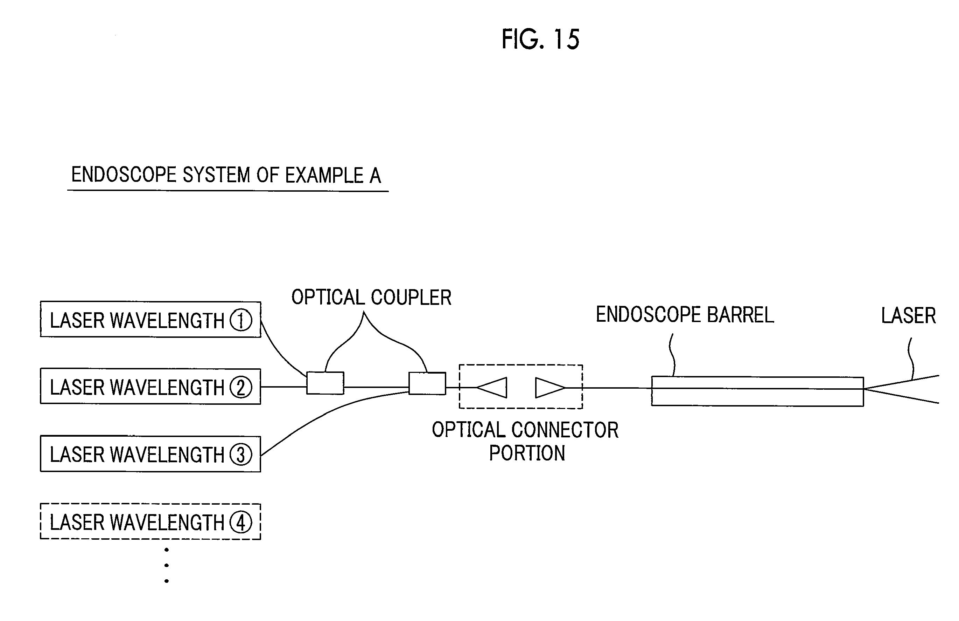

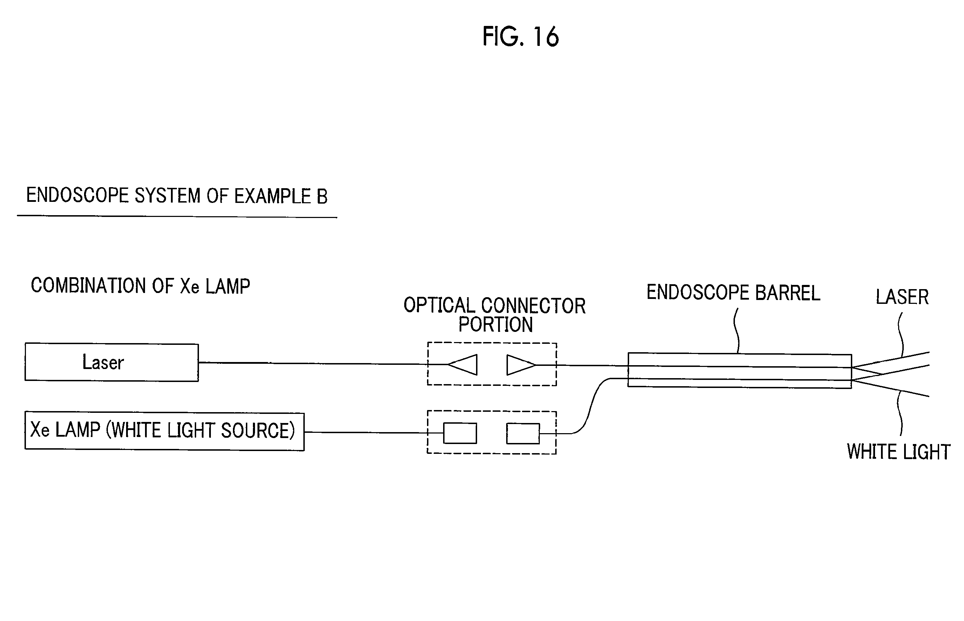

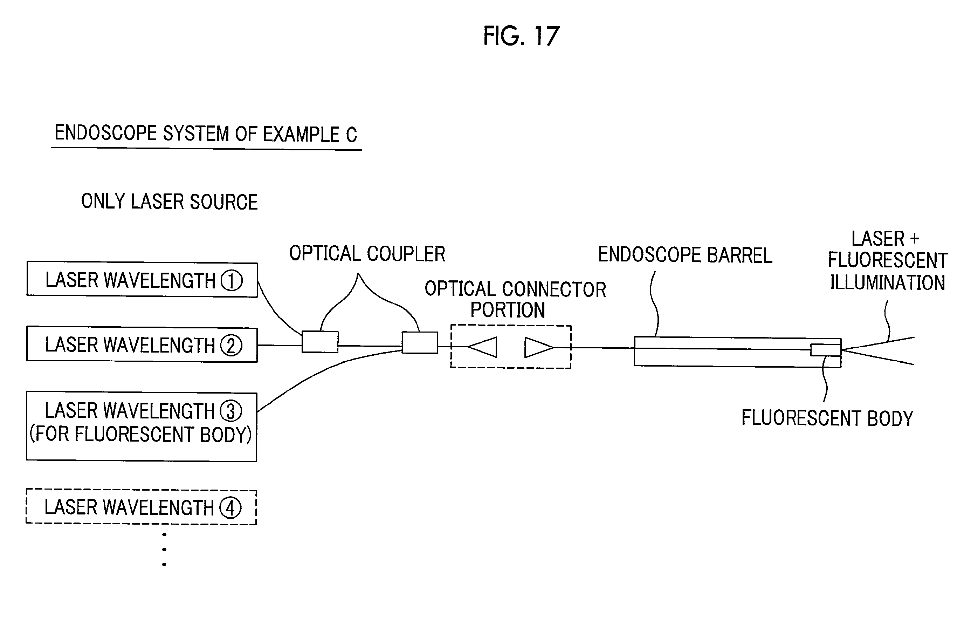

Fig. 15 is a diagram illustrating an endoscope system of Example A where only a laser source is used as an illumination light source. Fig. 16 is a diagram illustrating an endoscope system of Example B in which both a laser source and a xenon lamp are used as an illumination light source. Fig. 17 is a diagram illustrating an endoscope system of Example C where only a laser source is used as an illumination light source, but a fluorescent illumination laser source is included.

As shown in Fig. 15, in the endoscope system of Example A, the combined light flux which is formed by combining the respective light fluxes having different wavelengths and output from the laser source through an optical coupler is transmitted to the endoscope body through the optically coupled optical connector of the present invention, and the combined light flux (the laser) is irradiated from the endoscope body.

Further, as shown in Fig. 16, in the endoscope system of Example B, the light flux output from the laser source is transmitted to the endoscope body through the optical connector of the present invention, the light flux (white light) output from the xenon lamp is transmitted to the endoscope body through a general connector (bundle fiber), and then the respective light fluxes are individually irradiated from the endoscope body.

Further, as shown in Fig. 17, in the endoscope system of Example C, the combined light flux which is formed by combining the light flux output from plural laser sources with the light flux output from the fluorescent illumination laser source through an optical coupler is transmitted to the endoscope body through the optically coupled optical connector of the present invention, and the combined light flux (the laser) is irradiated from the endoscope body.

Furthermore, the present invention is not limited to the embodiment and the respective examples described above, and various modifications thereof may be made within the spirit of the present invention.

**Claims**

1. An optical connector comprising:

   a step-index-type light source side optical fiber that is disposed on a light source side and includes a core portion with a constant refractive index; and a step-index-type light receiving side optical fiber that is disposed on a light receiving side and includes a core portion with a constant refractive index, with both optical fibers optically coupled to each other by disposing end surfaces of the light source side optical fiber and the light receiving side optical fiber so as to face each other,
   wherein the light source side optical fiber and the light receiving side optical fiber are attachable to and detachable from each other, and
   wherein the light source side optical fiber has a taper portion in which the diameter of the core portion increases toward the end surface of the light source side optical fiber.

2. The optical connector according to claim 1,
   wherein the light receiving side optical fiber includes a taper portion in which the diameter of the core portion increases toward the end surface of the light receiving side optical fiber.

3. The optical connector according to claim 1,
   wherein the cross-section of the core portion of the light receiving side optical fiber has a same shape and size.

4. The optical connector according to any one of claims 1 to 3,
   wherein the end surface of the light source side optical fiber and the end surface of the light receiving side optical fiber are disposed so as to face each other in a non-contact state while both optical fibers are optically coupled to each other.

5. The optical connector according to any one of claims 1 to 4,
   wherein the optical connector is capable of passing a light there-through, with the light having at least two different types of wavelengths.

6. The optical connector according to any one of claims 1 to 4,
   wherein the optical connector is capable of passing a white light there-through

7. The optical connector according to any one of claims 1 to 6,
   wherein the area of the core portion in the end surface of the light receiving side optical fiber is larger than the area of the core portion in the end surface of the light source side optical fiber.

8. The optical connector according to any one of claims 1 to 6,
   wherein the end surface of the core portion of the light receiving side optical fiber and the end surface of the core portion of the light source side optical fiber are disposed so as to face each other without excess or insufficient overlap thereof in a state where both optical fibers are optically coupled to each other.

9. The optical connector according to claim 1,
   wherein the light source side optical fiber is in the range of the following conditional equation (1).

$$0.5 \leq L1/\varepsilon1 \ \dots \ (1)$$

   where L1 shows the length of the taper portion of the light source side optical fiber and $\varepsilon1$ shows the taper ratio of the light source side optical fiber.

10. The optical connector according to claim 2,
    wherein the light receiving side optical fiber is in the range of the following conditional equation (2).

$$0.5 \leq L2/\varepsilon2 \ \dots \ (2)$$

    where L2 shows the length of the taper portion of the light receiving side optical fiber and $\varepsilon2$ shows the taper ratio of the light receiving side optical fiber.

11. An endoscope system comprising:

    the optical connector according to any one of claims 1 to 10;
    a light source; and
    an endoscope body,
    wherein a light flux output from the light source is transmitted to the endoscope body through the optical connector so that the light flux is output from the endoscope body.

FIG. 1A

FIG. 1B

EP 2 500 755 A2

## FIG. 2A

100A

−Z

$\phi 1min$    θ1    $\phi 1max$    $\phi 2max$    $\phi 2min$    +Z

10   12   14   11   21   24   θ2   22   20

L1    d    L2

FIG. 2B

FIG. 2C

EP 2 500 755 A2

FIG. 2D

# FIG. 3

FIG. 4

# FIG. 5

HEATING AND STRETCHING

H

Ft

Ft

H

Ft

Ft

WELDING

CUTTING

H

Ft

POLISHING

# FIG. 6

FIG. 7A

Sh

Sh

FIG. 7B

Te

Te

FIG. 7C

Te

Sf

# FIG. 8

Legend:

□ BOTH SMALL-DIAMETER STRAIGHT OPTICAL FIBERS, WAVELENGTH OF 405 nm

■ BOTH SMALL-DIAMETER STRAIGHT OPTICAL FIBERS, WAVELENGTH OF 785 nm

△ BOTH TAPERED OPTICAL FIBERS, WAVELENGTH OF 405 nm

▲ BOTH TAPERED OPTICAL FIBERS, WAVELENGTH OF 785 nm

◇ TAPERED OPTICAL FIBER → LARGE-DIAMETER OPTICAL FIBER, WAVELENGTH OF 405 nm

◆ TAPERED OPTICAL FIBER → LARGE-DIAMETER OPTICAL FIBER, WAVELENGTH OF 785 nm

# FIG. 9

SIMULATION VALUE

TAPER LENGTH OF 1mm
TAPER LENGTH OF 2mm
TAPER LENGTH OF 10mm
TAPER LENGTH OF 80mm

# FIG. 10

MEASUREMENT VALUE (TAPER LENGTH OF 10 mm)

# FIG. 11

SIMULATION VALUE

FIG. 12

TAPER LENGTH L

CORE/CLAD DIAMETER OF OUTPUT
END SURFACE (230/250μm)

CORE/CLAD
DIAMETER(60/80μm)

# FIG. 13

FIG. 14

EP 2 500 755 A2

TAPER LENGTH L1 = 10mm
10mm

SEPARATION
DISTANCE d
=500 μm

TAPER LENGTH L2 = 10mm
10mm

CORE/CLAD DIAMETER
(60/80 μm)

# FIG. 15

## ENDOSCOPE SYSTEM OF EXAMPLE A

LASER WAVELENGTH ①

LASER WAVELENGTH ②

LASER WAVELENGTH ③

LASER WAVELENGTH ④

OPTICAL COUPLER

OPTICAL CONNECTOR PORTION

ENDOSCOPE BARREL

LASER

# FIG. 16

EP 2 500 755 A2

ENDOSCOPE SYSTEM OF EXAMPLE B

COMBINATION OF Xe LAMP

OPTICAL CONNECTOR
PORTION

ENDOSCOPE BARREL

LASER

| Laser |

| Xe LAMP (WHITE LIGHT SOURCE) |

WHITE LIGHT

# FIG. 17

EP 2 500 755 A2

ENDOSCOPE SYSTEM OF EXAMPLE C

ONLY LASER SOURCE

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006524845 A
- JP 2006309146 A